(19) Europäisches Patentamt
European Patent Office
Office européen des brevets

(11) Numéro de publication : **0 442 816 A1**

(12) **DEMANDE DE BREVET EUROPEEN**

(21) Numéro de dépôt : **91400380.1**

(22) Date de dépôt : **14.02.91**

(51) Int. Cl.[5] : **C07C 59/42,** C07C 57/03, C07C 51/09, C07C 51/367

(30) Priorité : **15.02.90 FR 9001814**

(43) Date de publication de la demande : **21.08.91 Bulletin 91/34**

(84) Etats contractants désignés : **AT BE CH DE DK ES FR GB GR IT LI LU NL SE**

(71) Demandeur : **RHONE-POULENC NUTRITION ANIMALE
Rue Marcel Lingot
F-03600 Commentry (FR)**

(72) Inventeur : **Gubelmann, Michel
39 Boulevard des Belges
F-69006 Lyon (FR)**

(74) Mandataire : **Savina, Jacques et al
RHONE-POULENC RORER S.A., Direction des Brevets, 20 avenue Raymond Aron
F-92160 Antony Cédex (FR)**

(54) **Nouveaux acides alpha-hydroxyles, procédé de préparation et leur utilisation.**

(57) La présente invention concerne de nouveaux acides α-hydroxylés de formule générale (I), leur procédé de préparation et leur utilisation.

En particulier, ces nouveaux composés permettent d'accéder, par décarboxylation oxydante, aux aldéhydes homologues inférieurs (prénal, citral).

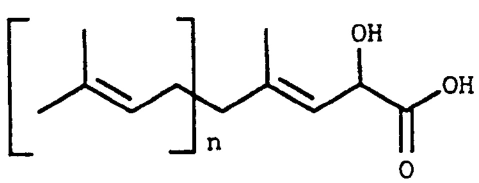

(I)

EP 0 442 816 A1

Jouve, 18, rue Saint-Denis, 75001 PARIS

# NOUVEAUX ACIDES α-HYDROXYLES, PROCEDE DE PREPARATION ET LEUR UTILISATION

La présente invention concerne de nouveaux acides carboxyliques α-hydroxylés β-insaturés. Plus particulièrement, elle concerne de nouveaux acides α-hydroxylés de formule générale :

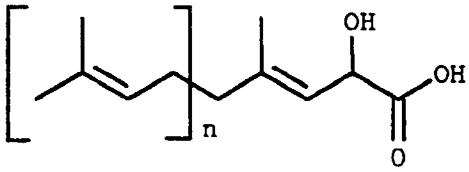

(I)

leur procédé de préparation et leur utilisation. En particulier, ces produits nouveaux constituent des intermédiaires permettant d'accéder, par décarboxylation oxydante, aux aldéhydes homologues inférieurs (prénal, citral ...). De tels aldéhydes permettent à leur tour d'accéder à la vitamine A, ou bien, peuvent être utilisés pour leurs propriétés aromatiques.

Il est connu par le brevet français 1 554 805 de préparer des composés carbonylés α-éthyléniques par isomérisation d'alcools α-acétyléniques. Cette isomérisation s'opère par chauffage de l'alcool, éventuellement dans un solvant, en présence de faibles quantités d'un catalyseur à base d'un métal appartenant aux colonnes 3b à 7b de la classification périodique. Avantageusement, cette isomérisation a lieu en phase liquide et le catalyseur est un dérivé minéral ou organique d'un métal choisi dans le groupe constitué par le vanadium, le niobium, le molybdène, le tungstène et le rhénium.

Il est également connu du brevet américain 3 057 888 de préparer des aldéhydes insaturés à partir d'esters de l'alcool propargylique 1,1-disubstitué par chauffage en milieu acide, en présence d'un catalyseur contenant un métal appartenant au groupe 1b de la classification périodique.

Il est encore connu des brevets américains 2 524 865 et 2 524 866 de préparer des aldéhydes éthyléniques par traitement d'alkynols en phase vapeur, sous l'action de différents catalyseurs acides. Toutefois, ce procédé ne fournit qu'un mélange ternaire d'aldéhyde, de cétone et d'hydrocarbure éthyléniques.

La présente invention décrit une nouvelle voie d'accès à ces aldéhydes, et notamment au prénal et au citral, à partir d'esters d'acides carboxyliques β,γ-insaturés. Cette voie qui, en outre, donne de très bons rendements, a permis de mettre en évidence et d'isoler de nouveaux composés intermédiaires acides carboxyliques α-hydroxylés β-insaturés.

Un objet de la présente invention concerne donc des composés nouveaux ayant pour formule générale (I)

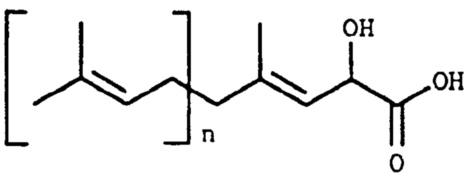

(I)

dans laquelle n peut être 0, 1, 2 ou 3.

En particulier, l'invention a pour objet les composés de formule générale (I) dans laquelle n = 0, c'est-à-dire l'acide hydroxy-2 méthyl-4 pentène-3 oïque, et n = 1, c'est-à-dire l'acide hydroxy-2 diméthyl-4,8 nonadiène-3,7 oïque.

Un autre objet de la présente invention concerne un procédé de préparation de ces composés, à partir d'esters d'acides carboxyliques β,γ-insaturés de formule générale (II), selon lequel :

– dans une première étape, on saponifie un ester de formule générale (II)

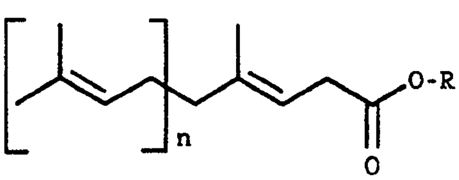

(II)

dans laquelle R est un groupement alkyl contenant 1 à 4 atomes de carbone et n est égal à 0, 1, 2, ou 3,

pour former l'acide correspondant,

– dans une deuxième étape, on prépare le dianion de l'acide ainsi obtenu par action d'une base choisie parmi les hydrure ou les amidure alcalins, et les alkyles d'organométalliques, éventuellement déposée ou greffée sur un support, en solvant organique,

– dans une troisième étape, on effectue l'oxygénation du dianion pour former l'acide de formule (I).

Plus particulièrement, la première étape de ce procédé met en jeu la réaction suivante : Saponification de l'ester (II) pour générer l'acide de formule générale (III)

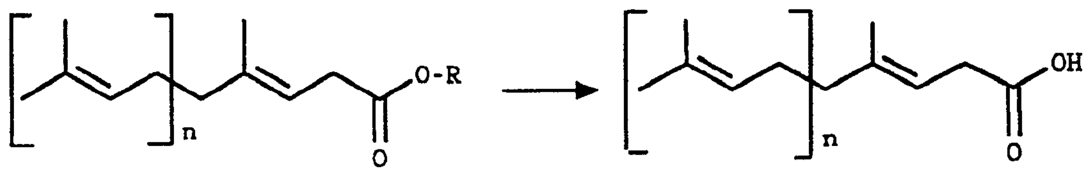

(II) (III)

Dans un mode de réalisation particulier de l'invention, R, qui peut être un groupement alkyl contenant de préférence de 1 à 4 atomes de carbone, est un groupement méthyl, et n, qui peut être 0, 1, 2 ou 3, est égal à 0 (méthyl-4 pentène-3 oate de méthyle) ou égal à 1 (diméthyl-4,8 nonadiène-3,7 oate de méthyle).

La réaction de saponification peut être effectuée au moyen d'une base forte de type M-OH, dans laquelle M est préférentiellement un métal alcalin ou un ammonium quaternaire, en solvant organique. On peut notamment travailler dans les solvants miscibles à l'eau, tels que les alcools (méthanol, éthanol, isopropanol...). Dans un mode particulier de réalisation de l'invention, on utilisera la soude méthanolique.

La température de la réaction est de préférence comprise entre la température ambiante et la température de reflux du mélange.

Les esters de formule générale (II) peuvent être préparés à partir de l'isoprène, ou d'un homologue supérieur, selon le procédé décrit dans le brevet français FR 81 01205. La réaction est la suivante :

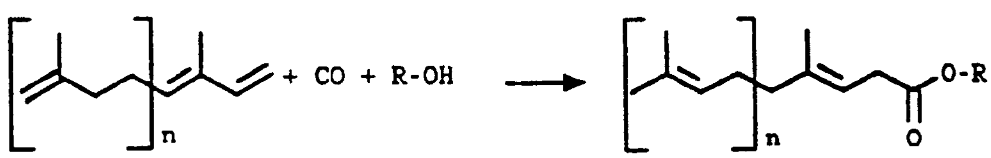

(II)

Cette réaction de carbonylation est effectuée au moyen d'oxyde de carbone, en présence de l'alcool correspondant à l'ester recherché, d'un hydracide halogéné (l'acide chlorhydrique ou l'acide bromhydrique notamment), d'un catalyseur au palladium (palladium métal, oxyde de palladium, sel ou complexe de palladium dont l'anion coordiné au cation palladium est une base "dure" ou "intermédiaire"), et d'un sel d'onium quatemaire d'un élément du groupe Vb choisi parmi l'azote, le phosphore, et l'arsenic, à une température comprise entre 50 et 150°C, et sous une pression d'oxyde de carbone de 50 à 300 bars. Dans ces conditions, on obtient l'ester avec de très bon rendements.

En ce qui concerne la deuxième étape du procédé : la préparation du dianion de l'acide (III), on peut procéder de la manière suivante :

Le dianion est obtenu par action d'une base telle que les hydrure ou les amidure alcalins, ou les alkyles d'organométalliques, éventuellement déposée ou greffée sur un support, en solvant organique. Selon la présente invention, l'amidure alcalin peut être choisi parmi le diisopropylamidure de lithium (LDA) qui peut être préparé "in situ" par action du butyllithium sur la diisopropylamine, le tertiobutylate, ou encore l'amidure de sodium. Parmi les hydrures utilisables dans cette réaction, on peut citer les hydrures de sodium, de potassium ou de calcium. En ce qui concerne les alkyles d'organométalliques, on peut utiliser les organolithiens, les organomagnésiens, ou les alkyles de sodium ou de potassium. On peut mentionner plus particulièrement le butyllithium.

Dans un mode de réalisation préféré de l'invention, on utilise comme base le LDA.

Dans le cas de bases hétérogènes, on peut utiliser en particulier les supports de type oxydes, tels que

notamment les alumines. A cet égard on peut mentionner comme bases hétérogènes le tertiobutylate de potassium ou le fluorure de potassium sur alumine.

Concernant le solvant organique, tous les solvants de type éther conviennent dans cette réaction. De préférence, on utilise le tétrahydrofuranne, le diisopropyléther, le méthyltertiobutyléther ou encore le paradioxanne. Dans un mode de réalisation préféré, on utilise le tétrahydrofuranne.

Enfin, la réaction de formation du dianion est avantageusement conduite à des températures basses, et notamment, des températures comprises entre -20°C et +20°C, et de préference, entre -10°C et +10°C. Dans certains cas, la température peut être provisoirement élevée en fin de réaction, pour déplacer le plus possible l'équilibre de la réaction vers le dianion. Ce traitement thermique de finition se fait par des températures comprises entre 20°C et 50°C, et, de préference, entre 30°C et 40°C.

La troisième étape qui concerne l'oxygénation du dianion, est effectuée de préférence de la manière suivante :

L'oxygénation du dianion pour générer l'α-hydroxyacide de formule générale (I) est réalisée au moyen d'oxygène ou d'air, éventuellement enrichi en oxygène, et de préférence d'air. Elle peut être effectuée en laissant en contact l'oxygène ou l'air avec la solution agitée du dianion, ou en balayant en surface ladite solution. Dans un autre mode de réalisation, l'oxygénation peut être obtenue au moyen d'air sous pression. Avantageusement, cette réaction est conduite après avoir amené la température à des valeurs proches de la température ambiante.

Lors de la mise en oeuvre du procédé objet de l'invention, des produits secondaires, dans lesquels l'oxygénation ne s'est pas produite en α de l'acide, peuvent se former. Cependant, ces produits sont très largement minoritaires, et, en raison de leur faible taux de cristallisation, peuvent être éliminés par filtration après cristallisation des produits principaux.

Un autre objet de la présente invention concerne l'utilisation de ces composés nouveaux pour la préparation des aldéhydes homologues inférieurs par décarboxylation oxydante au moyen d'un agent oxydant. Le schéma réactionnel est le suivant :

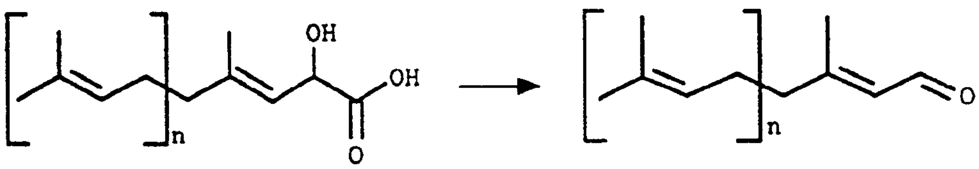

Cette réaction peut être effectuée au moyen d'un agent oxydant, tel que l'acétate d'un ou plusieurs métaux choisis parmi le cobalt, le manganèse, le plomb, l'argent, ou le couple cuivre-plomb, et de préférence parmi le cobalt et le plomb. En particulier, le tétracétate de plomb donne de très bon résultats. Il est également possible d'effectuer la décarboxylation oxydante par le biais des techniques radicalaires décrites par O. Toussaint, P. Capdeveille et M. Maumy (Tetrahedron Letters, Vol. 25, N° 35, 1984, p 3819). Notamment, on peut utiliser des métaux, tels qu'en particulier Cu2O, dans un solvant stabilisant le cuivre (I), tel que l'acétonitrile, à la pression atmosphérique.

D'autres objets et avantages de la présente invention apparaîtront à la lecture des exemples qui suivent et qui doivent être considérés comme illustratifs et non limitatifs.

EXEMPLE 1 - Synthèse de l'acide méthyl-4 pentène-3 oïque par saponification du méthyl-4 pentène-3 oate de méthyle

On utilise 12,8 g (100 mmoles) de l'ester que l'on dilue dans 50 ml de méthanol. 15 ml de soude 30 % (112 mmoles) sont additionnés goutte à goutte. La solution obtenue est chauffée à reflux pendant 3 heures sous agitation. On évapore ensuite le solvant, à sec, et le carboxylate résiduel est traité par 15 ml d'HCl concentré. L'acide est alors extrait à l'éther et séché sur $Na_2SO_4$. On évapore ensuite l'éther et on tire sous pompe à palettes pour éliminer les dernières traces d'eau. Dans ces conditions, le taux de transformation de l'ester est de 100 %, et le rendement de 92 %.

EXEMPLE 2 - Préparation de l'acide hydroxy-2 méthyl-4 pentène-3 oïque

Dans un réacteur tricol de 250 cm3 muni d'une agitation centrale, d'un réfrigérant, d'une ampoule de coulée de 50 cm3, d'une arrivée de gaz et d'un système de chauffage, on introduit, sous atmosphère d'argon, 4 g de diisopropylamine en solution dans 60 cm3 de tétrahydrofuranne anhydre. On refroidit par un bain de glace puis

on ajoute goutte à goutte en maintenant la température inférieure à 5°C, 37 cm3 d'une solution de butyllithium 1,1M dans l'hexane. Après 30 minutes d'agitation à une température de 2°C, on ajoute une solution de 2,28 g d'acide méthyl-4 pentène-3 oïque dans 30 cm3 de tétrahydrofuranne anhydre. Après agitation pendant 30 minutes, le mélange réactionnel est chauffé à 40°C pendant 1 heure.

Après refroidissement à une température voisine de 20°C, on introduit de l'air pendant 3 heures au moyen d'une baudruche tout en agitant fortement. La réaction est suivie par dosage des acides par résonance magnétique nucléaire du proton à 360 MHz.

Lorsque la réaction est terminée, on ajoute 2 fois 250 cm3 d'eau.

La phase aqueuse, séparée par décantation, est concentrée puis acidifiée par de l'acide chlorhydrique concentré et enfin extraite à l'éther.

Les phases éthérées, après séchage et concentration, fournissent une huile qui cristallise lentement par refroidissement.

Le produit brut obtenu contient 90 % d'acide hydroxy-2 méthyl-4 pentène-3 oïque et 10 % d'acide hydroxy-4 méthyl-4 pentène-3 oïque.

L'acide hydroxy-2 méthyl-4 pentène-3 oïque est séparé par filtration sur verre fritté.

L'acide hydroxy-2 méthyl-4 pentène-3 oïque présente les caractères physico-chimiques suivants :

– point de fusion : 95-98°C

– analyse élémentaire : C % calculé : 55,37 trouvé : 54,76

H % calculé : 7,75 trouvé : 7,28

– spectre infra-rouge (comprimé en mélange avec KBr) : bandes caractéristiques à : 3400 $cm^{-1}$ (OH alcool), 3100-2300 $cm^{-1}$ (OH acide), 2980 $cm^{-1}$ ($CH_3$), 1705 $cm^{-1}$ (C=O, acide) et 1070 $cm^{-1}$ (C=O, alcool)

– spectre de résonance magnétique nucléaire du proton (360 MHz, $CDCl_3$ , déplacements chimiques en ppm par rapport à l'hexaméthyldisilane pris comme référence) : 5,13 (d, 1H, =CH-) ; 4,85 (d, 1H, =CH-CH(OH)- ; 1,72 (2s, 6H, 2 x $CH_3$)

– spectre de masse (m/e) : $M^+$ = 130.

Le taux de transformation de l'acide méthyl-4 pentène-3 oïque est de 58 %.

EXEMPLE 3 -

On opère comme dans l'exemple 1 mais en effectuant un balayage d'air en surface.

Le taux de transformation de l'acide méthyl-4 pentène-3 oïque est de 100 %.

L'acide hydroxy-2 méthyl-4 pentène-3 oïque est isolé avec un rendement de 87 %.

On obtient l'acide hydroxy-4 méthyl-4 pentène-3 oïque avec un rendement de 5 %.

EXEMPLE 4 - Préparation du prénal à partir de l'α-hydroxyacide

Dans un ballon de 50 cm3 muni d'une agitation magnétique, on introduit 0,143 g d'acide hydroxy-2 méthyl-4 pentène-3 oïque, 5 cm3 d'une solution aqueuse contenant 90 % d'acide acétique et 0,54 g de tétracétate de plomb. On agite pendant 1 heure à 25°C. On ajoute 5 cm3 d'acide sulfurique 0,35M puis on sépare le sulfate de plomb précipité par filtration. Le prénal est précipité quantitativement dans le filtrat sous forme de dinitro-2,4 phénylhydrazone.

Le rendement est de 70,3 %.

EXEMPLE 5

Dans un ballon de 50 cm3 muni d'une agitation magnétique, on introduit 0,143 g d'acide hydroxy-2 méthyl-4 pentène-3 oïque, 5 cm3 de dichloro-1,2 benzène et, par petites fractions, 0,54 g de tétracétate de plomb. On agite pendant 1 heure à 25°C. Après décantation, l'analyse du mélange réactionnel par chromatographie en phase gazeuse montre que le taux de transformation de l'acide hydroxy-2 méthyl-4 pentène-3 oïque est de 100 % et que le rendement en prénal est de 86 %.

**Revendications**

1 - Nouveaux composés caractérisés par la formule générale :

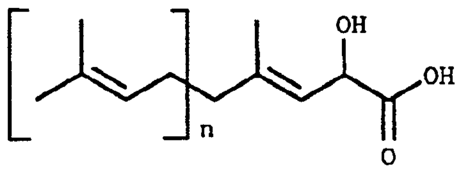

(I)

dans laquelle n est égal à 0, 1, 2 ou 3.

**2** - Composé selon la revendication 1 caractérisé en ce que, dans la formule (I), n = 0 ou n = 1.

**3** - Composé selon la revendication 2 caractérisé en ce qu'il s'agit de l'acide hydroxy-2 méthyl-4 pentène-3 oïque.

**4** - Procédé de préparation d'un composé selon l'une des revendications 1 à 3 caractérisé en ce que l'on effectue les étapes suivantes :

– dans une première étape, on saponifie un ester de formule générale :

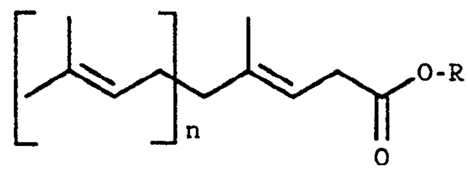

(II)

dans laquelle R est un groupement alkyl contenant 1 à 4 atomes de carbone et n peut être 0, 1, 2 ou 3, pour former l'acide correspondant,

– dans une deuxième étape, on prépare le dianion de l'acide ainsi obtenu par action d'une base choisie parmi les hydrure ou les amidure alcalins, et les alkyles d'organometalliques, eventuellement déposée ou greffée sur un support, en solvant organique,

– dans un troisième étape, on effectue l'oxygénation du dianion pour former l'acide de formule (I).

**5** - Procédé selon la revendication 4 caractérisé en ce que la saponification est effectuée au moyen d'une base forte de type M-OH dans laquelle M peut être un metal alcalin ou un ammonium quaternaire.

**6** - Procédé selon la revendication 5 caractérisé en ce que la saponification est effectuée au moyen de soude méthanolique.

**7** - Procédé selon la revendication 4 caractérisé en ce que l'amidure alcalin est choisi parmi le diisopropylamidure de lithium (LDA), le tertiobutylate, et l'amidure de sodium.

**8** - Procédé selon la revendication 7 caractérisé en ce que l'amidure alcalin est le LDA.

**9** - Procédé selon la revendication 7 caractérisé en ce que l'alkyle d'organometallique est choisi parmi les organolithiens, les organomagnésiens, les alkyles de potassium et de sodium.

**10** - Procédé selon la revendication 4 caractérisé en ce que l'hydrure est choisi parmi l'hydrure de sodium, l'hydrure de potassium et l'hydrure de calcium.

**11** - Procédé selon les revendications 4, et 7 à 9 caractérisé en ce que la base est déposée ou greffée sur un support de type oxyde, tel que les alumines.

**12** - Procédé selon les revendications 4, et 7 à 11 caractérisé en ce que le solvant est un solvant de type éther, choisi de préférence parmi le tétrahydrofuranne (THF), le diisopropyléther, le méthyltertiobutyléther ou le paradioxanne.

**13** - Procédé selon la revendication 12 caractérisé en ce que le solvant organique est le THF.

**14** - Procédé selon la revendication 4 caractérisé en ce que l'oxygénation du dianion est effectuée au moyen d'oxygène ou d'air, eventuellement enrichi en oxygène, et de préférence d'air.

**15** - Procédé selon l'une des revendications 4 à 14 caractérisé en ce que le dianion est formé à une température comprise entre -20°C et +20°C, et de preference entre -10°C et +10°C.

**16** - Procédé selon l'une des revendications 4 à 15 caractérisé en ce que dans la formule générale (II) R est un groupement méthyl, n = 0 et l'acide formé est l'acide hydroxy-2 méthyl-4 pentène-3 oïque.

**17** - Utilisation des composés de formule générale (I) selon les revendications 1 à 3 pour la préparation des aldéhydes homologues inférieurs par décarboxylation oxydante au moyen d'un agent oxydant.

**18** - Utilisation selon la revendication 17 caractérisée en ce que l'agent oxydant est un acétate d'un ou plusieurs metaux choisis parmi le cobalt, le manganèse, le plomb, l'argent, ou le couple cuivre-plomb, et de preference, parmi le cobalt et le plomb.

Office européen des brevets

# RAPPORT DE RECHERCHE EUROPEENNE

Numero de la demande

EP 91 40 0380

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | BULLETIN DE LA SOCIETE CHIMIQUE DE FRANCE, 1957, pages 658-662; J. COLONGE et al.: "No 108. - Etude sur les trichlorométhycarbinols secondaires saturés et éthyléniques. II. Action des réactifs alcalins" * Page 659, colonne 2, alinéa: "I - Alcools trichlorés éthyléniques" * --- | 1-3 | C 07 C 59/42<br>C 07 C 57/03<br>C 07 C 51/09<br>C 07 C 51/367 |
| D,A | FR-A-2 498 594 (RHONE-POULENC) * Page 17, ligne 7 * ----- | 4 | |
| | | | **DOMAINES TECHNIQUES RECHERCHES (Int. Cl.5)** |
| | | | C 07 C 59/00<br>C 07 C 69/00 |

Le présent rapport a été établi pour toutes les revendications

| Lieu de la recherche | Date d'achèvement de la recherche | Examinateur |
|---|---|---|
| LA HAYE | 14-05-1991 | KLAG M.J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)